(19) Europäisches Patentamt / European Patent Office / Office européen des brevets

(11) **EP 3 798 684 B1**

(12) **EUROPÄISCHE PATENTSCHRIFT**

(45) Veröffentlichungstag und Bekanntmachung des Hinweises auf die Patenterteilung:
**21.08.2024   Patentblatt 2024/34**

(21) Anmeldenummer: **19199855.8**

(22) Anmeldetag: **26.09.2019**

(51) Internationale Patentklassifikation (IPC):
**G01T 1/24** *(2006.01)*      **G01T 7/00** *(2006.01)*

(52) Gemeinsame Patentklassifikation (CPC):
**G01T 7/005; G01T 1/24**

(54) **DATENKORREKTUR IN DER RÖNTGENBILDGEBUNG**

DATA CORRECTION IN X-RAY IMAGING

CORRECTION DE DONNÉES DANS L'IMAGERIE PAR RAYONS X

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**31.03.2021   Patentblatt 2021/13**

(73) Patentinhaber: **Siemens Healthineers AG**
**91301 Forchheim (DE)**

(72) Erfinder:
  • **Baer-Beck, Matthias**
    **91054 Erlangen (DE)**
  • **Hillenbrand, Achim**
    **91052 Erlangen (DE)**
  • **Hupfer, Martin**
    **91052 Erlangen (DE)**
  • **Petersilka, Martin**
    **91325 Adelsdorf (DE)**
  • **Raupach, Rainer**
    **91336 Heroldsbach (DE)**
  • **Stierstorfer, Karl**
    **91052 Erlangen (DE)**

(74) Vertreter: **Siemens Healthineers**
**Patent Attorneys**
**Postfach 22 16 34**
**80506 München (DE)**

(56) Entgegenhaltungen:
**WO-A2-2006/094493     US-A1- 2017 224 299**

• **J. CAMMIN ET AL: "Compensation of nonlinear distortions in photon-counting spectral CT: deadtime loss, spectral response, and beam hardening effects", PROCEEDINGS OF SPIE, vol. 8313, 23 February 2012 (2012-02-23), pages 83131T - 83131T-7, XP055084909, ISSN: 0277-786X, DOI: 10.1117/12.911217**
• **TKACZYK, J.E. ET AL: "Contrast-to-Noise of a Non-Ideal, Multi-bin, Photon Counting X-ray Detector", SPIE, PO BOX 10 BELLINGHAM WA 98227-0010, USA, vol. 7961, 2011, XP040556163**
• **KAPPLER, S. ET AL: "Quantum-counting CT in the regime of count-rate paralysis: introduction of the pile-up trigger method", SPIE, PO BOX 10 BELLINGHAM WA 98227-0010, USA, vol. 7961, 2011, pages 1 - 10, XP040556068**

EP 3 798 684 B1

**Beschreibung**

Eine neuartige Technologie im Bereich der Röntgenbildgebung, insbesondere der Computertomographie, sind direkt wandelnde bzw. direkt-konvertierende oder auch (Photonen) zählende Detektoren, die eine spektral aufgelöste bzw. energieselektive Bildgebung ermöglichen. Zählende Röntgendetektoren verwenden keinen Szintillator, der Röntgenphotonen zunächst in optisches Licht umwandelt, sondern einen Halbleiter, der Röntgenphotonen direkt detektiert. Vorteilhaft lösen direkt-konvertierende Detektoren neben dem Ort auch die Energie eines einfallenden Photons auf, bspw. in der spektralen Computertomographie. Dabei werden auf die Detektorfläche einfallende Röntgenquanten je nach Quantenenergie bestimmten Energiebändern bzw. Bins zugeordnet, d.h. energieselektiv klassifiziert und jeweils die Ereignisse pro Pixel pro Energieband gezählt. Zählende Röntgendetektoren eignen sich folglich zur gleichzeitigen Erzeugung von wenigstens zwei Projektionsdatensätzen, die sich in ihrer Röntgenquantenenergieverteilung unterscheiden bei Verwendung nur einer Röntgenstrahlungsquelle. Typisch sind zwei bis vier Energiebänder. Die wenigstens zwei Projektionsdatensätze werden jeweils durch sämtliche Pixel-Signale des Detektors innerhalb eines Energiebandes gebildet. Als Detektormaterialien für quantenzählende Detektoren eignen sich insbesondere die Halbleiter Cadmium-Tellurid, Cadmium-Zink-Tellurid oder Gallium-Arsenid oder, im Falle eines Flachdetektors, amorphes Selen oder dergleichen. Die Schwellwerte der Energiebänder werden vorab Hersteller-seitig festgelegt und die Elektronik im Detektormodul entsprechend eingestellt. Es können entweder feste, unveränderliche Schwellwerte voreingestellt werden oder die Schwellwerte können in Abhängigkeit eines bzw. zusammen mit einem gewünschten Scanprotokoll, bspw. für eine Kopf- oder Thorax-Aufnahme, Benutzerseitig ausgewählt werden. Das heißt, die Schwellwerte können, um den medizinisch relevanten Informationsgehalt einer erfassten Bildaufnahme zu optimieren, passend zum Anfangsverdacht, zur antizipierten Diagnose oder zur medizinischen Fragestellung angepasst werden.

Insbesondere bei Photonen zählenden Röntgendetektoren besteht das Problem, dass durch Pile-Up-Effekte im Detektor starke Nichtlinearitäten bestehen, die wiederum durch spektrale Effekte wie etwa eine Strahlaufhärtung beeinflusst werden.

Pile-Up beschreibt den Fall, in dem an einem Röntgendetektor mindestens zwei Detektionsereignisse innerhalb einer Zeit erfolgen, die kürzer als die Auflösungszeit für einen bzw. die Dauer eines elektrischen Impulses ist, der durch ein einzelnes einfallendes Röntgenquant erzeugt wird. Liegen die Detektionsereignisse zeitlich sehr nah bei einander, werden die Detektionsereignisse zu einem Impuls mit aufaddierten Amplituden entsprechend ihrer Quantenenergie zusammengefasst (peak pile-up). Erfolgen die Detektionsereignisse nun in zeitlich größerem Abstand, können sie zwar als eigenständige Ereignisse detektiert werden, jedoch zumindest der/die zeitlich später einfallende(n) Röntgenquanten wird/werden mit einer zu höheren Werten hin verfälschten Quantenenergie erfasst (tail pile-up). Bei einem Pile-Up kann folglich ein Zählverlust von Detektionsereignissen ähnlich wie durch Totzeit resultieren. Zudem wird eine Verschiebung des Röntgenquantenspektrums hin zu höheren Quantenenergien hervorgerufen. In Abhängigkeit der zuvor festgelegten Energie-Bins können durch Pile-Up überlagerte Detektionsereignisse in falsche Bins eingruppiert und damit die Zählrate mehrerer Projektionsdatensätze verfälscht werden. Pile-Up-Effekte hängen sehr stark von der Zähl-Rate eines Systems ab, je höher also die Zählrate, umso größer wird der Einfluss von Pile-Up-Effekten höherer Ordnung (Überlagerung von mehr als zwei Detektionsereignisses).

Ein Strahlaufhärtungseffekt ist eine Verschiebung der effektiven Energie eines Röntgenstrahls hin zu einem höheren Wert bei Durchdringen von Materie. Dies liegt an der Energieabhängigkeit des linearen Röntgenschwächungskoeffizienten begründet. Mit zunehmender Durchdringungstiefe erhöht sich also die mittlere Energie der Röntgenquanten, weil Röntgenquanten geringerer Energien bevorzugt absorbiert werden. Der relative Anteil niederenergetischer Röntgenquanten wird mit zunehmendem Materialdurchtritt immer kleiner. Je größer also die Materialdicke, umso stärker äußert sich der Aufhärtungsfehler in den erfassten Detektorsignalen.

Nichtlinearitäten infolge von Pile-Up und Strahlaufhärtung äußern sich beide in einer Verschiebung der effektiven Röntgenstrahlenenergie hin zu einem höheren Wert. Dadurch lassen sie sich nicht ohne weiteres trennen.

Bislang wurde dieses Problem iterativ adressiert, indem wiederholte Korrekturschritte, also zunächst eine Nichtlinearitätskorrektur, dann eine Strahlaufhärtungskorrektur, dann eine Nichtlinearitätskorrektur und so weiter durchgeführt wurden, wodurch das Problem zumindest näherungsweise gelöst werden konnte. Das beschriebene Prozedere ist jedoch langwierig und rechenintensiv. Aus WO 2006/094493 und US 2017/224299 sind Verfahren zur Korrektur von Strahlaufhärtung und Nichtlinearitäten bekannt, bei denen getrennte Korrekturfunktionen für beide Effekte bestimmt und verwendet werden.

Demgegenüber ist es Aufgabe der vorliegenden Erfindung, alternative Mittel bereit zu stellen, die es erlauben, zuverlässig, automatisch und schnell eine Fehlerkorrektur an energieselektiven Messdaten durchzuführen. Insbesondere ist es Aufgabe der vorliegenden Erfindung, eine Fehlerkorrektur für wenigstes zwei Datenfehler gleichzeitig bereit zu stellen, die sich gegenseitig beeinflussen.

Diese Aufgabe wird gelöst durch ein computerimplementiertes Verfahren zur Korrektur eines Eingabedatensatzes, entsprechende Recheneinheit, entsprechendes Computerprogramm und entsprechenden computerlesbaren Medium gemäß den unabhängigen Ansprüchen. Bevorzugte vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein computerimplementiertes Verfahren zur Korrektur eines Eingabedatensatzes. Das Verfahren umfasst eine Vielzahl von Schritten.

Ein erster Schritt betrifft ein Erfassen eines Eingabedatensatzes umfassend wenigstens einen Datenfehler. Ein weiterer Schritt betrifft ein Bestimmen einer Korrekturfunktion. Ein weiterer Schritt betrifft ein Erzeugen eines korrigierten Ausgabedatensatzes durch Anwenden der Korrekturfunktion auf den Eingabedatensatz.

Ein weiterer Schritt betrifft ein Ausgeben des korrigierten Ausgabedatensatzes.

Die Erfindung zeichnet sich aus durch die Ausbildung der Korrekturfunktion, die eine Reduzierung von wenigstens zwei, sich gegenseitig beeinflussenden Datenfehlern in dem Eingabedatensatz bewirkt. Mit anderen Worten können mittels der hier vorgeschlagenen Korrekturfunktion parallel, d.h. in einem Rechenschritt, zwei Datenfehler minimiert bzw. behoben werden, die eine Verfälschung des Eingabedatensatzes verursachen.

Die gegenseitige Beeinflussung der wenigstens zwei Datenfehler besteht zumindest darin, dass sich verschiedene Datenfehler in ähnlicher Weise als Fehlereffekte auf den Eingabedatensatz auswirken, sodass sich Anteile der Datenverfälschung nicht mehr eindeutig auf einen der beiden Datenfehler zurückführen lassen.

Das erfindungsgemäße Verfahren wandelt unter Anwendung der erfindungsgemäßen Korrekturfunktion einen Eingabedatensatz in einen Ausgabedatensatz um. Mit anderen Worten entspricht die Korrekturfunktion einer mathematischen Vorschrift, die einen Eingabedatensatz in einen Ausgabedatensatz überführt.

Während der Eingabedatensatz Datenfehler aufweist, ist der Ausgabedatensatz von diesen Datenfehlern befreit. Zumindest sind die Datenfehler im Ausgabedatensatz deutlich reduziert.

Der Eingabedatensatz ist dadurch gekennzeichnet, dass er Fehlereffekte wenigstens basierend auf einem, bevorzugt basierend auf mindestens zwei verschiedenen Datenfehlern umfasst.

Ein erfindungsgemäßer, Eingabedatensatz entspricht Projektionsdaten eines Röntgendetektors, wie er bei einer medizinischen Untersuchung eines Patienten, bspw. einer Computertomographie-Untersuchung entsteht. Insofern ist der Eingabedatensatz insbesondere ein medizinischer Eingabedatensatz. Insbesondere umfasst der Eingabedatensatz wenigstens zwei Projektionsdatensätze, die sich spektral, also in ihren (mittleren) Röntgenquantenenergien unterscheiden. Ein Projektionsdatensatz ist dabei für das über ihn abgebildete Quantenenergiespektrum repräsentativ für eine räumliche Intensitätsverteilung einfallender Röntgenquanten über die sensitive Detektorfläche hinweg, wobei die detektierten Röntgenquanten ausgehend von der Röntgenstrahlenquelle ein Untersuchungsobjekt passiert haben. Jeder Pixeleintrag, also Projektionsmesswert des Projektionsdatensatzes entspricht folglich einer Anzahl Röntgenquanten, die in diesem Pixel mit einer Quantenenergie entsprechend dem jeweiligen Energiebereich, Energieband bzw. Energie-Bin eingefallen sind. Der Eingabedatensatz entspricht folglich einer fehlerbehafteten, detektierten Schwächungsverteilung des abgebildeten Untersuchungsobjektes.

Mit anderen Worten umfasst der Schritt des Erfassens des Eingabedatensatzes eine (medizinische) Messdatenaufnahme mittels Röntgenstrahlenquelle und Röntgenstrahlendetektor sowie das Übertragen der Messdaten in eine Recheneinheit für eine dortige Weiterverarbeitung.

Ein Untersuchungsobjekt im Sinne der Erfindung ist ein Patient, wobei es sich meist um einen Menschen handelt. Grundsätzlich kann der Patient auch ein Tier sein. Das Untersuchungsobjekt kann aber auch als nicht-lebender, insbesondere ein nicht-medizinischer Gegenstand sein, bspw. eine Pathologische Probe, eine mechanisches Bauteil oder aber ein historisches Artefakt. Im Folgenden werden daher die beiden Begriffe "Untersuchungsobjekt" und "Patient" synonym verwendet. Ein erfindungsgemäßer, Ausgabedatensatz entspricht einem Eingabedatensatz, der von umfassten Datenfehlern bereinigt wurde. Das heißt, auch der Ausgabedatensatz insbesondere als medizinischer Ausgabedatensatz ausgebildet, der bspw. als Projektionsdatensatz eines Röntgendetektors zu verstehen ist, der, wie der Eingabedatensatz insbesondere wenigstens zwei spektral unterschiedliche Projektionsmessdatensätze umfassen kann. Der Ausgabedatensatz entspricht folglich einer korrigierten, detektierten Schwächungsverteilung des abgebildeten Untersuchungsobjektes.

Der Schritt des Ausgebens des Ausgabedatensatzes umfasst bevorzugt eine Übertragung des Ausgabedatensatzes und eine Recheneinheit bzw. Rekonstruktionseinheit, die eingerichtet ist, mittels einer an sich bekannten Rekonstruktionsvorschrift wie bspw. einer (iterativen oder gewichteten) gefilterten Rückprojektion oder einer algebraischen Rekonstruktion im Wesentlichen Artefakt-freie Röntgen-Bilddaten zu erzeugen.

Erfindungsgemäß ist das Bestimmen der Korrekturfunktion den Schritten des Erfassens, des Erzeugens und des Ausgebens vorgelagert.

Erfindungsgemäß ist die Korrekturfunktion ausgebildet, wenigstens Datenfehler basierend auf einem während der Erfassung des Eingabedatensatzes auftretenden Pile-Up-Effekt und Strahlaufhärtungseffekt in dem Eingabedatensatz zu reduzieren. Diese bereits eingangs erläuterten zwei Datenfehler verursachenden Effekte beeinflussen sich gegenseitig, da sie jeweils unter anderem eine Verschiebung des detektierten Röntgenquantenenergiespektrums zu höheren Werten hin verursachen, sodass nicht mehr feststellbar ist, welcher Datenfehleranteil auf welchen der beiden Effekte zurückzuführen ist. Die erfindungsgemäße Korrekturfunktion erübrigt vorteilhaft eine Zuordnung von Datenfehleranteilen zu den verursachenden Effekten und korrigierte diese gemeinsam, wodurch Rechenzeit eingespart und Arbeitsabläufe effektiviert werden können.

In einer anderen Ausführung des erfindungsgemäßen Verfahrens ist die Korrekturfunktion ferner ausgebildet, einen weiteren Datenfehler basierend auf einem Streustrahlungseffekt in dem Eingabedatensatz zu reduzieren. Der Streustrahlungseffekt bezeichnet das Entstehen von gestreuten Röntgenquanten, die bei einer Wechselwirkung mit durchstrahltem Material ihre Strahlungsrichtung ändern. Gestreute Röntgenquanten treffen, wenn überhaupt, in einem nicht ihrer ursprünglichen Strahlungsrichtung entsprechenden Detektorpixel auf und verursachen so eine Verfälschung der räumlichen Röntgenstrahlungsintensitätsverteilung. Darüber hinaus verlieren Röntgenquanten durch ein Streuungsphänomen einen Teil ihrer Quantenenergie, sodass ein gestreutes Röntgenquant einem falschen Energieband zugeordnet werden kann. Die Streustrahlungsintensität hängt von der Materialdicke sowie der Art des Materials ab. Streustrahlung kann insbesondere durch eine Mehrfachstreuung entstehende Strahlung, also Streustrahlung höherer Ordnung umfassen. Streustrahlung kann ferner auch als Querstreuung eines Dual-Energy-Röntgenbildgebungssystems ausgebildet sein. Mit anderen Worten entspricht die Streustrahlung derjenigen Strahlung die von einer Röntgenstrahlenquelle eines ersten Strahler-Detektor-Paares ausgesandt, jedoch so gestreut wurde, dass sie von dem Röntgendetektor eines zweiten Strahler-Detektor-Paares des Systems detektiert wird und/oder umgekehrt. Die erfindungsgemäße Korrekturfunktion erübrigt vorteilhaft eine weitere Zuordnung von Datenfehleranteilen zu den verursachenden Effekten und korrigiert diese effektiv gemeinsam.

Die nun folgenden Ausführungen des erfindungsgemäßen Verfahrens können allein oder in Kombination geeignet sein, ein Verfahren zur Bestimmung einer Korrekturfunktion anzugeben, wobei die erfindungsgemäße Korrekturfunktion eine Reduzierung von wenigstens zwei, sich gegenseitig beeinflussenden Datenfehlern in dem Eingabedatensatz bewirkt.

In einer anderen Ausführung des erfindungsgemäßen Verfahrens ist die Korrekturfunktion als eine von einem Monitorwert bezüglich der für die Erfassung des Eingabedatensatzes verwendeten Röntgenstrahlenquelle abhängige Funktion ausgebildet. Der Monitorwert ist ausgebildet, ein Maß für die applizierte Röntgenstrahlung anzugeben. Der Monitorwert ist bevorzugt als der an der Röntgenstrahlenquelle eingestellte Röntgenröhrenstrom ausgebildet, der maßgeblich die Intensität des mittels Röntgenröhre ausgesandten Röntgenstrahlbündels bestimmt. Der Monitorwert kann auch ein Dosiswert sein. Mit anderen Worten ist die Korrekturfunktion ausgebildet, eine Röhrenstrom-bzw. Röntgenstrahlungsintensitäts-abhängige Korrektur des Eingabedatensatzes auszuführen. Dies ist besonders vorteilhaft, da es die Intensitäts- bzw. Zählratenabhängigkeit von Datenfehlern Rechnung trägt.

In einer anderen Ausführung des erfindungsgemäßen Verfahrens umfasst ein Bestimmen der Korrekturfunktion eine Kalibriermessung mit einem spektral auflösenden Röntgendetektor.

Ein spektral auflösender Röntgendetektor ist ausgebildet, einen Messdatensatz zu erzeugen, der wenigstens zwei sich spektral unterscheidende Projektionsdatensätze umfasst. Mit anderen Worten ist ein spektral auflösender Röntgendetektor ein energieselektiver Röntgendetektor. Ein spektral auflösender Röntgendetektor kann als quantenzählender Röntgendetektor ausgebildet sein, wie eingangs schon beschrieben. Er kann aber auch als Zwei-Schicht-Detektor ausgebildet sein.

Ein Zwei-Schicht-Detektor oder auch Dual oder Double Layer Detektor ist ausgestaltet, das einfallende Röntgenröhrenspektrum in einen niederenergetischen und einen hochenergetischen Anteil zu zerlegen. Dazu ist der Zwei-Schicht-Detektor aus zwei Schichten aufgebaut. Eine der Röntgenstrahlungsquelle zugewandte Detektorschicht misst Röntgenphotonen der einfallenden Röntgenstrahlung mit niedriger Energie und weist die gemessenen Signale einem ersten Projektionsmessdatensatz zu. Diese erste Detektorschicht wird von hochenergetischer Röntgenstrahlung durchdrungen. Röntgenphotonen mit höherer Quantenenergie werden in der darunter bzw. dahinter, also von der Röntgenstrahlungsquelle abgewandten Detektorschicht gemessen und einem zweiten Projektionsmessdatensatz zugeordnet. Typischerweise umfassen beide Detektorschichten einen Szintillator, folglich handelt es sich bei einem Zwei-Schicht-Detektor typischerweise um einen indirekt konvertierenden Detektor. Als Szintillationsmaterial kommen Kristalle wie Cäsium-Jodid, Cadmium-Wolframat oder keramische Stoffe, wie beispielsweise Gadoliniumoxysulfid oder dergleichen zum Einsatz.

Mit anderen Worten werden in dieser Ausführung spektral aufgelöste Kalibrierdaten umfassend wenigstens zwei spektral unterschiedliche Kalibrierdatensätze erzeugt, die in die Bestimmung der Korrekturfunktion eingehen.

In einer bevorzugten Ausführung des erfindungsgemäßen Verfahrens umfasst die Kalibriermessung ein Erfassen von Kalibrierdaten eines Testobjektes umfassend wenigstens zwei Basismaterialien. Mit anderen Worten wird in dieser Ausführung ein Kalibrierdatensatz umfassend wenigstens zwei spektral unterschiedliche Kalibrierdatensätze erzeugt, wovon jeder eine Röntgenschwächungsverteilung des Testobjektes bezüglich seines Energie-Bins beschreibt. Das Testobjekt, im Wesentlichen ein Phantom, ist aus wenigstens zwei Basismaterialien aufgebaut. Das Testobjekt kann alternativ aus zwei Testobjekten, jeweils umfassend nur ein Basismaterial aufgebaut sein.

Damit eignet sich die Kalibrierdaten vorteilhaft für eine Basismaterialzerlegung.

Bei einer Basismaterialzerlegung wird von der Überlegung ausgegangen, dass ein mittels Röntgendetektor gemessener Röntgenschwächungswert als Linearkombination bzw. Linienintegral entlang eines Strahlpfads von Röntgenschwächungswerten der Basismaterialien bezüglich der Röntgenquantenenergieverteilung eines jeden Energiebands beschrieben werden kann. Gemessene Röntgenschwächungswerte bzw. -verteilungen ergeben sich aus den wenigstens zwei Kalibrierdatensätzen zu jeweils unterschiedlichen Röntgenquantenenergieverteilungen. Material bzw. Basismaterial kann jeder Stoff oder jedes beliebige Gewebe sein, insbesondere können Wasser, Kontrastmittel wie Jod, Weichteilgewebe, Knochen und dergleichen in Frage kommen. Es können im Rahmen der Erfindung auch mehr als zwei Basismaterialien kombiniert werden.

Insofern umfassen die Basismaterialien in einer bevorzugten Ausführung des erfindungsgemäßen Verfahrens eine der folgenden Materialkombinationen:

- Wasser - Iod,
- Waser - Knochen, oder
- Aluminium - Eisen.

Die erfindungsgemäße Kalibriermessung umfasst vorteilhaft mehrere Messungen mit mehreren Testobjektion, die sich in die der Dicke der umfassten Basismaterialien unterscheiden. Bspw. können mehrere Testobjekte mit einer Materialkombination, bspw. Wasser und Knochen gemessen werden, wobei die Dicke eines der Basismaterialien weiter zu- und die Dicke des zweiten Basismaterials im gleichen Maß auch zu oder abnimmt.

Für jedes Dickenverhältnis der Materialkombination werden zudem mehrere Messungen mit unterschiedlichem Monitorwert, insbesondere unterschiedlichem Röhrenstrom der Röntgenstrahlenquelle durchgeführt.

Derart werden für die gewählte Materialkombination und verschiedene Dickenverhältnisse der Materialien eine Reihe Kalibrierdatensätze erzeugt, die eine Energie-, Dicke- und Röhrenstrom-abhängige Röntgenabschwächungsverteilung der Basismaterialien darstellen. Mit anderen Worten kann für Kalibrierdatensatz-Paare/Gruppen jeweils umfassend zwei Kalibrierdatensätze aus zwei oder mehr Energiebändern des Röntgendetektors eine Basismaterialzerlegung durchgeführt.

Das Röntgenabschwächungsverhalten eines Basismaterials ist in der Theorie bekannt und steht bspw. in Form von Tabellen zum Abruf zur Verfügung. Insofern ist auch für jede beliebige Kombination aus Basismaterialien und beliebige Dickeverhältnisse derselben und verschiedene Monitorwerte ein zu erwartender, also idealer Schwächungsdatensatz bekannt bzw. bestimmbar.

Die vorliegende Erfindung basiert nun auf der Überlegung, dass die wie beschrieben erzeugten Kalibrierdaten bezüglich des Testobjektes schon die beschriebenen Datenfehler aufweisen und folglich die Kalibrierdatensätze von dem theoretisch bekannten bzw. idealen Röntgenabschwächungsverhalten abweichen. Erfindungsgemäß wird nun die Korrekturfunktion als diejenige vom Monitorwert abhängige Funktion bestimmt, die die Kalibrierdatensätze auf die idealen Projektionsdatensätze abbildet.

Die Korrekturfunktion kann als Polynom, als LinearKombination mehrerer Funktionen oder als nicht-lineare Funktion ausgebildet sein. Insbesondere eine Linearkombination hat den Vorteil, dass die Optimierung mathematisch sehr einfach und direkt analytisch durchführbar ist, d.h. Koeffizienten lassen sich dabei direkt und nicht iterativ ausrechnen.Je nach Ausgestaltung der Korrekturfunktion erfolgt basierend auf den Kalibrierdatensätzen und den idealen Projektionsdatensätzen eine Parametrierung der Korrekturfunktion.

In einer weiteren Ausführung des erfindungsgemäßen Verfahrens umfasst die Kalibriermessung eines Testobjektes eine Berücksichtigung wenigstens zweier integrierte Wirkungsquerschnitte von Compton-Effekt und Photoeffekt. Diese Ausführung stellt eine Alternative zu der vorherigen Ausführung der Erfindung dar. Anstelle einen Röntgenabschwächungskoeffizienten in individuelle Abschwächungskoeffizienten zweier oder mehrerer Basismaterialien zu zerlegen, kann er auch Wechselwirkungsspezifisch in einen Compton-Abschwächungskoeffizienten und einen Photo-Abschwächungskoeffizienten zerlegt werden, welche sich analog zur Basismaterialzerlegung durch Inversion zweier spektral unterschiedlicher Kalibrierdatensätze ermitteln lassen. Die theoretischen/idealen Abschwächungskoeffizienten sind für verschiedene Materialien und verschiedene Monitorwerte bekannt. Das Testobjekt kann in dieser Ausführung auch nur ein Material umfassen. Beim Compton-Effekt gibt ein Röntgenquant bei einem elastischen Zusammenstoß mit einem Elektron einen Teil seiner kinetischen Energie unter Richtungsänderung ab. Beim Photoeffekt wird das Röntgenquant von dem Elektron vollständig absorbiert.

In einer weiteren Ausführung des erfindungsgemäßen Verfahrens umfasst das Bestimmen der Korrekturfunktion eine Kalibriermessung eines Testobjektes berücksichtigend wenigstens zwei monochromatische Wirkungsquerschnitte. Diese Ausführung stellt eine weitere Alternative zu den vorherigen Ausführungen der Erfindung dar. Anstelle einen Röntgenabschwächungskoeffizienten in individuelle Abschwächungskoeffizienten zweier oder mehrerer Basismaterialien zu zerlegen, kann er auch energieselektiv in Abschwächungskoeffizienten für spezielle Quantenenergiewerte zerlegt werden, welche sich ebenfalls durch Inversion zweier spektral unterschiedlicher Kalibrierdatensätze ermitteln lassen. Die theoretischen/idealen Abschwächungskoeffizienten sind erneut für verschiedene Materialien und verschiedene Monitorwerte bekannt. Das Testobjekt kann auch in dieser Ausführung nur ein Material umfassen.

In einer weiteren, alternativen Ausführung der Erfindung umfasst ein Bestimmen der Korrekturfunktion eine Simulation einer Kalibriermessung in einer der oben beschriebenen Ausführung. Hier werden die Kalibrierdatensätze, sprich Kalibriermesswerte jedes Pixelelements des Röntgendetektors, rein rechnerisch ermittelt, indem mittels einer Simulationsvorschrift das Detektionsverhalten für jedes einzelne Energieband jedes einzelnen Detektor-Pixels monitorwert-abhängig für ein definiertes Testobjekt simuliert wird. Die Simulation generiert bevorzugt Datenfehler in den simulierten Kalibriermesswerten, wie sie durch Pile-Up Effekte oder eine Strahlaufhärtung hervor gerufen würden. Besonders bevorzugt generiert die Simulation zusätzlich einen Datenfehler, wie er durch Streustrahlung hervorgerufen würde.

In einer weiteren Ausführung des erfindungsgemäßen Verfahrens ist die Korrekturfunktion als eine mittels maschinellem Lernen trainierte Funktion ausgebildet. Maschinelles Lernen im Sinne der Erfindung umfasst eine computer-implementierte Technik, bei der ein Algorithmus auf Basis von bestehenden Daten Muster bzw. Gesetzmäßigkeiten erkennt und unter Anwendung derselben in Bezug auf unbekannte, neue Daten eigenständig Lösungen ableitet. Voraussetzung für eine eigenständige Lösungsfindung ist eine Trainingsphase, in der ein Algorithmus des Maschinen-Lernens auf einen bekannten, definierten und zumeist sehr großen Datenbestand angewandt wird, um diejenigen Regeln bzw. Vorhersagen zu finden, die eine gewünschte Ausgabe bzw. ein gewünschtes Ergebnis erzielen. Das Training kann als überwachtes oder unüberwachtes Training ausgebildet sein, wobei in der ersten Variante dem Algorithmus Werte-Paare in Form von Eingabewerten und dazu gehörigen, korrekten Ausgabewerten präsentiert werden, wohingegen in der zweiten Variante der Algorithmus sich basierend auf den Eingabewerten eigenständig derart selber anpassen muss, dass er die korrekten Ausgabewerte liefert.

Besonders vorteilhaft ist der Algorithmus des Maschinen-Lernens als künstliches neuronales Netz ausgebildet. Ein künstliches neuronales Netz umfasst zwischen einer Eingabe- und einer Ausgabeschicht bevorzugt eine Vielzahl von weiteren Schichten jeweils umfassend wenigstens einen Knoten. Jeder Knoten entspricht dabei einer Verarbeitungseinheit. Knoten innerhalb einer Schicht des Netzes können über gerichtete Verbindungen (edges) mit Knoten anderer Schichten verbunden sein. Die Verbindungen definieren den Datenfluss innerhalb des Netzes. Jeder Knoten repräsentiert folglich eine Operation, die auf die Eingabedaten angewendet wird. Ferner verfügt jeder Knoten bzw. jede seiner Verbindungen über einen Gewichtungsparameter (weight). Über diesen Gewichtungsparameter wird der Einfluss bzw. die Wichtigkeit der Ausgabe eines Knotens als Eingabewert für einen Empfängerknoten definiert. In der Trainingsphase, die bevorzugt als überwachtes Lernen ausgeführt wird, ,lernt' das künstliche Neuronale Netz anhand der Trainingsdaten die Gewichtungsparameter für alle Knoten bzw. Verbindungen und passt diese solange an, bis die Ausgabeschicht des Netzes die korrekten Ausgabewerte liefert.

Eine trainierte Funktion des Maschinen-Lernens stellt also im Rahmen seines Trainings einen festen Zusammenhang zwischen Eingabewerten (Input), hier in Form der Kalibrierdatensätze und den dazu gehörigen Monitorwerten, und Ausgabewerten (Output) in Form von idealen Projektionsdatensätzen herstellt. Die trainierte Funktion ersetzt in dieser Ausführung die Korrekturfunktion.

Die Erfindung betrifft in einem weiteren Aspekt eine Recheneinheit zur Korrektur eines Eingabedatensatzes gemäß Anspruch 11.

Die Korrekturfunktion ist ausgebildet, eine Reduzierung von wenigstens zwei, sich gegenseitig beeinflussenden Datenfehlern in dem Eingabedatensatz zu bewirken.

Die Recheneinheit kann insbesondere als Recheneinheit einer medizinischen Bildgebungsanlage ausgebildet sein, umfassend eine Röntgenstrahlenquelle, einen energieselektiven Röntgendetektor und eine Patientenliege. Die Recheneinheit kann auch als die Recheneinheit eines energieselektiven Röntgenstrahlendetektors ausgebildet sein. In einer bevorzugten Ausführung ist die Recheneinheit mit der medizinischen Bildgebungsanlage umfassend den energieselektiven Röntgendetektor verbunden. Alternativ ist die Recheneinheit mit dem energieselektiven Röntgendetektor direkt verbunden. Vorteilhaft ist die Recheneinheit in die medizinische Bildgebungsanlage integriert. Alternativ kann die Recheneinheit auch entfernt bzw. abgelegen davon angeordnet sein. Die Recheneinheit kann ausgebildet sein, insbesondere den Schritt des Bestimmens der Korrekturfunktion durchzuführen, aber auch das gesamte erfindungsgemäße Verfahren, für eine medizinische Bildgebungsanlage oder für eine Vielzahl von Anlagen durchzuführen, z.B. in einem Radiologie-Zentrum oder Krankenhaus umfassend mehrere medizinische Bildgebungsanlagen.

Ein weiterer Aspekt der vorliegenden Erfindung betrifft einen Röntgendetektor in Form eines energieselektiven Röntgendetektors, umfassend die erfindungsgemäße Recheneinheit.

Die Erfindung betrifft ferner ein Computerprogramm mit Programmcode, um das erfindungsgemäße Verfahren zur Korrektur eines Eingabedatensatzes durchzuführen, wenn das Computerprogramm auf einem Computer ausgeführt wird.

Die Erfindung betrifft ferner ein computerlesbares Medium mit Programmcode eines Computerprogramms, um das erfindungsgemäße Verfahren zur Korrektur eines Eingabedatensatzes, wenn das Computerprogramm auf einem Computer ausgeführt wird.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:

FIG 1 eine schematische Darstellung des erfindungsgemäßen Verfahrens in einem Ausführungsbeispiel der vorliegenden Erfindung,

FIG 2 eine schematische Darstellung eines neuronalen Netzes zur Verwendung in einem erfindungsgemäßen Verfahren,

FIG 3 einen erfindungsgemäßen Röntgendetektor gemäß einem Ausführungsbeispiel der vorliegenden Erfindung, und

FIG 4 eine medizinische Bildgebungsanlage in Form eines Computertomographen umfassend eine erfindungsgemäße Recheneinheit gemäß einem Ausführungsbeispiel der vorliegenden Erfindung.

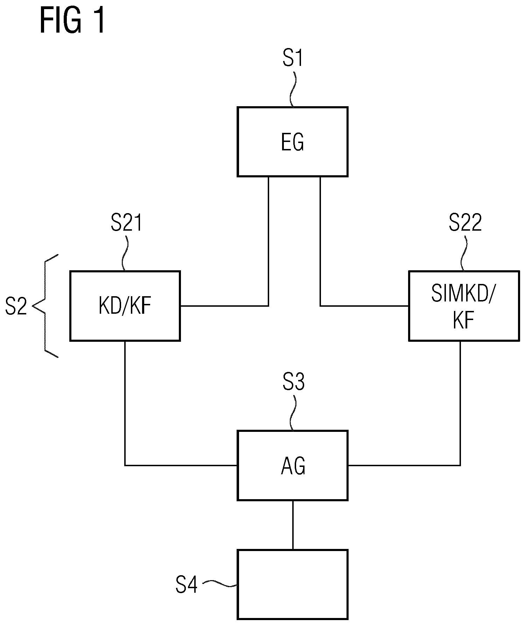

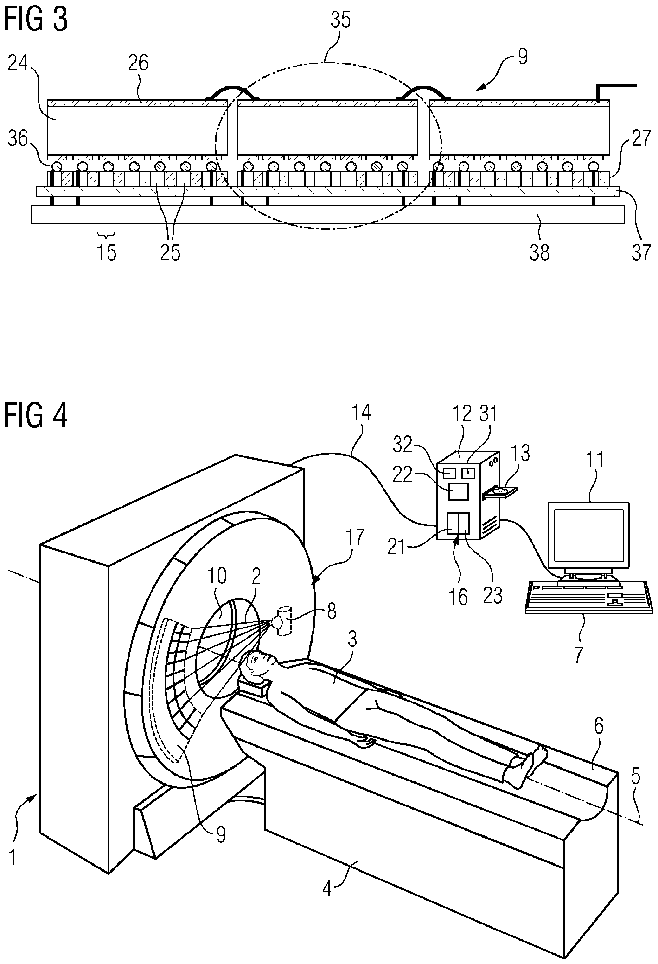

Figur 1 zeigt eine schematische Darstellung des erfindungsgemäßen Verfahrens in einem Ausführungsbeispiel. In einem ersten Schritt S1 erfolgt ein Erfassen von medizinischen Eingabedaten bzw. eines medizinischen Eingabedatensatzes EG. Das Erfassen kann eine Datenerfassung mit einer Bildgebungsanlage, insbesondere umfassend einem Röntgendetektor 9 gemäß Figur 3 oder 4 umfassen. Mit anderen Worten kann das Erfassen gemäß Schritt S1 das Erzeugen bzw. Generieren des medizinischen Eingabedatensatzes EG umfassen. Alternativ kann der medizinische Eingabedatensatz EG ein bereits erfasster uns bestehender Datensatz sein, der aus einer externen oder internen Speichereinheit 22 ausgelesen werden kann, bspw. aus einem Krankenhausinformationssystem umfassend elektronische Patientenakten. Das Erfassen des medizinischen Eingabedatensatzes kann allgemein auch ein Erfassen des Eingabedatensatzes EG mittels Schnittstelle 31 bzw. Datenverarbeitungseinheit 16 oder Korrektureinheit 23 der Bildgebungsanlage 1 umfassen, also den Schritt der Übertragung bzw. des Einlesens der Eingabedaten von einer der genannten Datenquellen 9, 22, etc. umfassen. Allgemein umfasst der Schritt S1 des Erfassens das Beschaffen des Eingabedatensatzes EG.

Das Erfassen gemäß Schritt S1 kann auch ein Erfassen desjenigen Monitorwertes m bezüglich der Röntgenquelle 8 umfassen, der für die Erfassung des Eingabedatensatzes EG an der Röntgenquelle 8 eingestellt war. Der Monitorwert m kann bspw. als Meta-Information in dem Eingabedatensatz EG selbst umfasst und mit diesem zusammen übertragen werden. Alternativ wird der Monitorwert m als eigenständige Größe, jedoch umfassend eine Relation bzw. Verknüpfung zum Eingabedatensatz EG, (zwischen-)gespeichert und übertragen. Der Monitorwert m stellt erfindungsgemäß ein Maß für die applizierte Röntgenstrahlung dar. In diesem Ausführungsbeispiels entspricht er dem Röhrenstrom der Röntgenquelle 8. Er kann aber auch einem Röntgendosiswert entsprechen.

Bei dem medizinischen Eingabedatensatz handelt es sich um Projektionsdaten, wie sie bspw. mit einem Röntgendetektor 9 erfasst werden können. Die Projektionsdaten beschreiben eine räumliche Verteilung der Strahlenintensität eines Röntgenstrahlenbündels, welches ausgehend von einer Röntgenquelle ein Untersuchungsobjekt passiert hat und dabei abhängend von der Beschaffenheit, Zusammensetzung und Dicke des passierten Gewebes abgeschwächt wurde. Um die räumliche Röntgenstrahlungsverteilung aufzulösen, erfasst der Röntgendetektor für eine Vielzahl von flächig angeordneten Pixelelementen die Anzahl der in diesem Pixelelement einfallenden Röntgenquanten. Die Projektionsdaten resultieren erfindungsgemäß aus einer Röntgenmessung mit einem energie-selektiven Röntgendetektor, bspw. in Form eines Zwei-Schicht-Detektors oder eines quantenzählenden Detektors. Energie-selektive Detektoren sind ausgebildet, für jedes Pixelelement ein, zwei oder mehr als zwei energie-selektive Messwerte zu liefern. Einfallende Röntgenquanten werden entsprechend ihrer Quantenenergie in einen der vorab eingestellten Energiebereiche klassifiziert. Ein Projektionsdatensatz umfasst die Gesamtheit aller Pixel-Messwerte eines Energiebereichs. Die Projektionsdaten des Eingabedatensatzes können also ein, zwei oder mehrere Projektionsdatensätze entsprechend der Anzahl der Energiebereiche umfassen. Sofern eine Datenerfassung eine Untersuchung des Untersuchungsobjektes in mehreren Projektionsrichtungen vorsieht, können die Projektionsdaten entsprechend der Anzahl der Projektionsrichtungen mehrere Projektionsdatensätze bezüglich jedes Energiebereichs umfassen.

Der medizinische Eingabedatensatz EG umfasst Datenfehler. Die Datenfehler beruhen auf mindestens den beiden Abbildungsfehlern Pile-Up und Strahlaufhärtung. Der medizinische Eingabedatensatz EG kann zudem auch noch Datenfehler in Form von Streustrahlungsfehlern aufweisen. Allen Datenfehlern ist gemein, dass sie sich gegenseitig beeinflussen, sodass eine Separierung der Datenfehleranteile entsprechend der verursachenden Effekte nicht oder nur mit großem Rechenaufwand möglich ist.

In einem zweiten Schritt S2 erfolgt das Bestimmen einer Korrekturfunktion KF. Die Korrekturfunktion KF wird derart bestimmt, dass sie eingerichtet ist, die genannten Datenfehler in dem Eingabedatensatz EG zu korrigieren. Die Korrekturfunktion KF ist ferner als eine von dem Monitorwert m abhängige Funktion ausgebildet. Schritt S2 wird bevorzugt von einer Bestimmungseinheit 21 einer Recheneinheit 12 durchgeführt. Schritt S2 wird erfindungsgemäß vor Schritt S1 durchgeführt.

Schritt S2 kann als eine von mehreren alternative Ausführungsformen S21, S22 ausgebildet sein. Gemäß Schritt S21 erfolgt das Bestimmen der Korrekturfunktion über eine Kalibriermessung eines Testobjektes. Gemäß Schritt S22 erfolgt das Bestimmen der Korrekturfunktion über eine Simulation einer Kalibriermessung. Besonders bevorzugt umfasst das Bestimmen der Korrekturfunktion ein Trainieren eines Algorithmus des Maschinen-Lernens.

Schritt S21 umfasst eine Kalibriermessung mit einem spektral auflösenden Röntgendetektor 9. Die Kalibriermessung wird mit einem Testobjekt umfassend zwei Basismaterialien, alternativ auch mehr Basismaterialien durchgeführt, bspw. dann wenn Materialien mit K-Kantenabsorption zu quantifizieren sind. Folgende Basismaterialkombinationen sind bevorzugt:

- Wasser - Iod,
- Wasser - Knochen,
- Aluminium - Eisen.

Ein Testobjekt mit einer ausgewählten Basismaterialkombination wird für eine Vielzahl von verschiedenen Dickeverhältnissen gemessen, bspw. in den Folgenden Verhältnissen:

| Dicke Material 1 (cm) | Dicke Material 2 (cm) |
| --- | --- |
| 0 | 1/2/3 |
| 5 | 0/1/2/3 |
| 10 | 0/1/2/3 |
| 20 | 0/1/2/3 |

und/oder

| Dicke Material 2 (cm) | Dicke Material 1 (cm) |
| --- | --- |
| 0 | 5/10/20 |
| 1 | 0/5/10/20 |
| 2 | 0/5/10/20 |
| 3 | 0/5/10/20 |

Das Testobjekt kann aus Testobjekten umfassend jeweils nur eine Basismaterial einer bestimmten Dicke zusammengesetzt werden.

Die Kalibriermessung kann für eine Vielzahl von Monitorwerten m durchgeführt werden. Ergebnis der Kalibriermessung sind Kalibrierdaten KD umfassend eine Vielzahl von Kalibrierdatensätzen für eine Vielzahl von Dickeverhältnissen. Jeder Kalibrierdatensatz umfasst Kalibriermesswerte si = [s1, ..., sx] für jedes Pixelelement, die spezifisch für jeweils einen der Energiebereiche des Röntgendetektors sind. Für jeden Monitorwert m und jedes Dickeverhältnis liegen mindestens zwei spektral unterschiedliche Kalibrierdatensätze entsprechend der Energiebereiche des Röntgendetektors vor.

Die Erfindung geht nun von der Überlegung aus, dass die Kalibriermesswerte si für jedes Pixelelement genauso durch Fehlereffekte wie Pile-Up, Strahlaufhärtung, etc. fehlerbehaftet sind, wie Messwerte von Projektionsdatensätzen. Jedoch ist für Basismaterialien das ideale, energieabhängige Schwächungsverhalten bekannt. Für jedes Dickeverhältnis einer Basismaterialkombination sind also für alle möglichen Röhrenströme m ideale Schwächungsdatensätze ji = [j1, ..., jx] bekannt bzw. können berechnet werden.

Die Erfindung nutzt nun die allgemein bekannten Annahme, dass ein gemessener (Röntgenschwächungs-)Messwert in die die Röntgenschwächung verursachenden Schwächungsanteile von Basismaterialien zerlegt werden kann und nutzt die Tatsache, dass für alle Konstellationen Monitorwert - Basismaterialzusammensetzung/Dickeverhältnis wenigstens zwei spektral unterschiedliche Kalibrierdatensätze für den Röntgendetektor vorliegen.

Für jeden Kalibrierdatensatz der Kalibierdaten wird nun Kalibriermesswert- also Pixelelement-weise eine Basismaterialzerlegung durchgeführt. Mit anderen Worten wird jeder Kalibriermesswert si als Funktion der Basismaterial-Linienintegrale $L = [L1, ..., Lp], p \leq n, n =$ Anzahl Basismaterialien, zu si = [m, L1, ..., Lp] dargestellt.

Auch die idealen Schwächungsmesswerte ji lassen sich als Funktion der Basismaterial-Linienintegrale L zu ji = [m, L1, ..., Lp] darstellen.

Die Korrekturfunktion KF wird nun für jeden Energiebereich so bestimmt, dass für jeden Energiebereich die Kalibriermesswerte si und den Monitorwert m auf die (idealen) Linienintegrale abbildet zu L = KF(s, m).

Die Korrekturfunktion KF kann in einer Ausführung ein Polynom in den Variablen si und m sein, welches den Zusammenhang zwischen den Größen s, m und L optimal wiedergibt. Die Korrekturfunktion Kf kann alternativ auch als Linearkombination von anderen Funktionen in si und m dargestellt werden. In beiden Fällen führt das Problem, als "least squares" Problem formuliert, auf ein lineares Gleichungssystem. Alternativ könnte die Korrekturfunktion auch als nichtlineare Funktion ausgebildet sein und derart angepasst werden, dass sie die Kalibriermesswerte optimal wiedergibt.

Anstelle der Kalibriermesswerte si könnten zunächst die logarithmischen Größen zu

$$\acute{L} := -\log\left(\frac{si}{m}\right) + \log\left(\frac{si0}{m0}\right)$$

berechnen, wobei die mit Index 0 gekennzeichneten Größen die bei einer Kalibriermessung des Röntgendetektors (z.B. frei Luft, also ohne Testobjekt) gewonnenen Kalibriermesswerte sind. Vorteil dieses Verfahrens ist, dass nach einer erneuten Kalibriermessung der Arbeitspunkt der Korrekturfunktion KF wiederhergestellt wird, sodass bspw. eine Luftmessung mit dem Luftkalibrierstrom sicher den Wert (:= 0 als Eingangswert für die Korrekturfunktion KF hätte.

Anstelle der logarithmischen Größe $\acute{L}$ könnten mit dergleichen Wirkung auch die de-logarithmierten Größen

$$\acute{I} := \frac{si}{m} \Big/ \frac{si0}{m0}$$

im Eingang der Korrekturfunktion KF verwendet werden. Alternativ könnten im Ausgang der Korrekturfunktion KF die de-logarithmierten Größen $I_a == \exp(-L_a)$: a=1, ..., p. verwendet werden.

In einer anderen Ausführung kann die Korrekturfunktion KF als eine mittels maschinellem Lernen trainierte Funktion, insbesondere als neuronales Netz 400, ausgebildet sein. Die Kalibriermesswerte si sowie die Monitorwerte m können in dieser Ausführung als Trainingseingangsdaten verwendet werden. Die alternativen Ausgestaltungen der Kalibriermesswerte (logarithmiert bzw. de-logarithmiert) könnten evtl. das Lernen der Korrekturfunktion KF durch das neuronales Netz 400 erleichtern. Das trainierte neuronale Netz könnte als Ausgabewerte erfindungsgemäß die Linienintegrale L, aber auch direkt die idealen Schwächungsmesswerte ji ausgeben.

Anstelle einer Basismaterialzerlegung kann zur Bestimmung der Korrekturfunktion KF eine Kalibriermessung eines Testobjektes berücksichtigend wenigstens zwei integrierte Wirkungsquerschnitte von Compton-Effekt und Photoeffekt umfasst. Diese Vorgehensweise beruht auf der Annahme, dass Röntgenschwächungen aller Materialien als Linearkombination der Wirkungsquerschnitte von Photoeffekt (Absorptionseffekt) und Comptoneffekt (Streuungseffekt) dargestellt werden können. Dies gilt zumindest näherungsweise und in Abwesenheit von K-KantenAbsorption. Analog könnten Polychromatische Röntgenschwächungsverteilungen auch als Linearkombination verschiedener monochromatischer Wirkungsquerschnitte dargestellt werden.

In einem alternativen Schritt S22 werden simulierte Kalibrierdatensätze SIMKD durch eine Simulation einer Kalibriermessung mittels Röntgendetektor erzeugt. Hier wird die Kalibrierdatenerfassung des Röntgendetektors in den verschiedenen in Bezug zu Schritt S21 erläuterten Konstellationen aus Basismaterial-Zusammensetzung und Dickeverhältnis und Monitorwert simuliert. In diesem Fall könnte zusätzlich ein Streuquantenfluss $\Phi$ mit bekanntem Spektrum in die Simulation einbezogen werden und die Kalibriermesswerte si auch als Funktion des Streuquantenflusses simuliert werden. Dann hätte die Korrekturfunktion KF als weiteren Eingangsparameter den Streuquantenfluss $\Phi$. Vorteilhaft könnte die Korrekturfunktion derart Datenfehler basierend auf Streustrahlung (die wiederum die spektralen bzw. Pileup-Verhältnisse des RÖntgendetektors ändert) berücksichtigen.

Eine Korrekturfunktion KF in Form eines neuronales Netzes müsste dann auch mit verschiedenen Werten von $\Phi$ trainiert werden.

In einem Schritt S3 erfolgt das Korrigieren des Eingabedatensatzes EG durch Anwenden der Korrekturfunktion KF auf denselben. Schritt S3 kann bevorzugt durch eine Korrektureinheit 23 einer Recheneinheit 12 durchgeführt werden. Dadurch wird der Ausgabedatensatz AG erzeugt. Die Korrekturfunktion befreit den medizinischen Eingabesatz durch Korrektur jedes einzelnen Projektionsmesswert um die darin enthaltenen Datenfehler bzw. reduziert diese.

In einer weiteren bevorzugten Ausgestaltung des Verfahrens kann Schritt S3 einen Prüfschritt umfassen, ob sich Metalle im Strahlengang befinden, die die Röntgenstrahlung sehr stark schwächen. In dieser Situation könnte die Korrekturfunktion KF unbrauchbare Werte liefern. Dazu könnte aus dem unkorrigierten Eingabedatensatz EG zunächst ein Röntgenbild rekonstruiert werden. Anhand des Röntgenbildes könnte eine an sich bekannte Metalldetektion durchgeführt werden. Detektierte Metallanteile des Bildes könnten vorwärts projeziert werden. Daraus würden sich Informationen ergeben, welcher Messstrahl Metall enthält. Diese zusätzliche Information könnte bevorzugt in die Korrekturfunktion KF einfließen.

In einem weiteren Schritt S4 wird der korrigierte Ausgabedatensatz AG bspw. an eine Rekonstruktionseinheit 32 zur Rekonstruktion von im Wesentlichen Artefakt-freien Röntgen-Bildern ausgegeben.

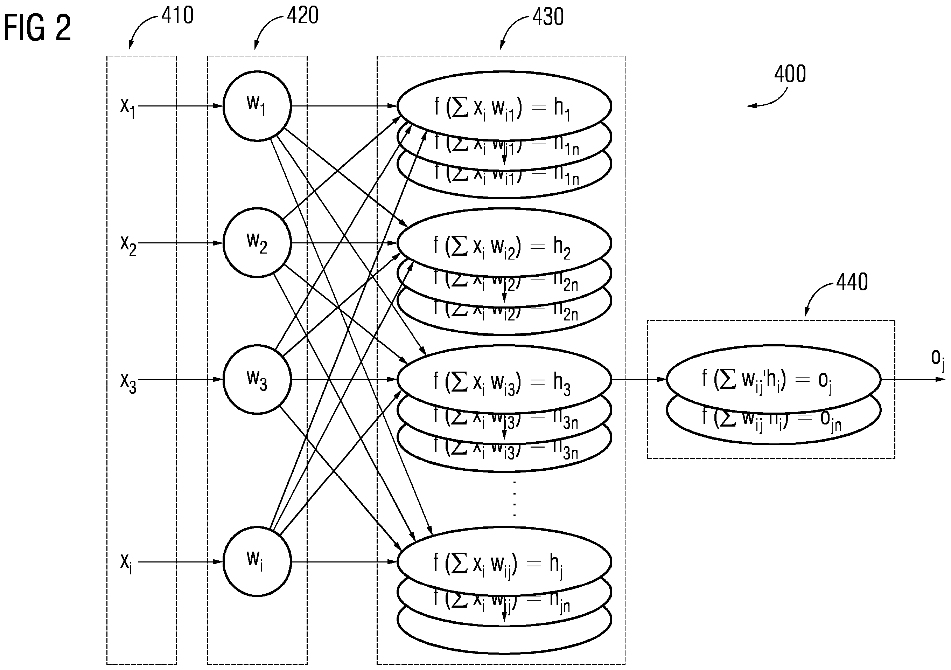

Figur 2 zeigt eine als künstliches neuronales Netz 400 ausgebildete Korrekturfunktion, wie sie im Verfahren gemäß Figur 1 zum Einsatz kommen kann. Das neuronale Netz 400 antwortet auf Eingabewerte zu einer Vielzahl von Eingangsknoten $x_i$ 410 die angewendet werden, um eine oder eine Vielzahl von Ausgaben $o_j$ zu erzeugen. Das neuronale Netz 400 lernt in diesem Ausführungsbeispiel, indem es die Gewichtungsfaktoren $w_i$ (weights) der einzelnen Knoten basierend auf Trainingsdaten anpasst. Mögliche Eingabewerte der Eingangsknoten $x_i$ 410 sind die Vielzahl von durch Pile-Up, Strahlaufhärtung, Streustrahlung, etc. fehlerbehafteten, energie-selektiven, Röntgenröhrenstrom-abhängigen Kalibriermesswerte si bezüglich eines Testobjektes und der dazu gehörige Röhrenstromwert m. Die Ausgabewerte 440 des neuronalen Netzes 400 entsprechen bevorzugt den entsprechenden idealen Schwächungsmessdaten ji ohne die durch Abbildungsfehler verursachten Datenfehler oder den Linienintegralen **L.** Die Ausgabe 440 kann über einen einzelnen oder eine Vielzahl von Ausgabeknoten $o_j$ erfolgen. Die Eingabewerte können bspw. konsekutiv für jeden Röhrenstromwert m in der Art eingegeben werde, dass jeder Eingangsknoten $x_i$ 410 den Messwert eines Detektorpixels entsprechend der Projektionsdatensätze empfängt. Entsprechend können die korrigierte Projektionsdatensätze pixelweise pro Ausgabeknoten ausgegeben werden.

Das künstliche neuronale Netz 400 umfasst bevorzugt eine versteckte Schicht 430, die eine Vielzahl von Knoten $h_j$ umfasst. Es können mehrere versteckte Schichten $h_{jn}$ vorgesehen sein, wobei eine versteckte Schicht 430 Ausgabewerte einer anderen versteckten Schicht 430 als Eingabewerte verwendet. Die Knoten einer versteckten Schicht 430 verrichten mathematische Operationen. Ein Ausgabewert eines Knotens $h_j$ entspricht dabei einer nicht-linearen Funktion f seiner Eingabewerte $x_i$ und der Gewichtungsfaktoren $w_i$. Nach dem Erhalt von Eingabewerten $x_i$, führt ein Knoten $h_j$ eine Summierung einer mit den Gewichtungsfaktoren $w_i$ gewichteten Multiplikation jedes Eingabewerts $x_i$ durch, wie durch folgende Funktion bestimmt:

$$h_j = f\left(\sum_i x_i \cdot w_{ij}\right)$$

Insbesondere wird ein Ausgabewert eines Knotens $h_j$ als Funktion f einer Knoten-Aktivierung, bspw. eine Sigmoidalfunktion oder eine lineare Rampenfunktion gebildet. Die Ausgabewerte $h_j$ werden an den bzw. die Ausgabeknoten $o_j$ übertragen. Erneut wird eine Summierung einer gewichteten Multiplikation jedes Ausgabewertes $h_j$ als Funktion der Knoten-Aktivierung f berechnet:

$$o_j = f\left(\sum_i h_i \cdot w'_{ij}\right)$$

Das hier gezeigte neuronale Netz 400 ist ein Feed-Forward Netz, bei dem alle Knoten 430 die Ausgabewerte einer vorherigen Schicht in Form ihrer gewichteten Summe als Eingabewerte verarbeiten. Selbstredend können erfindungsgemäß auch andere neuronale Netztypen zum Einsatz kommen, bspw. Feedback-Netze, bei denen ein Eingabewert eines Knotens $h_j$ gleichzeitig auch sein Ausgabewert sein kann.

Das neuronale Netz 400 wird mittels einer Methode des überwachten Lernens trainiert, um Muster zu erkennen. Eine bekannte Vorgehensweise ist die Back-Propagation, die für alle Ausführungsbeispiele der Erfindung angewandt werden kann. Während des Trainings wird das neuronale Netz 400 auf Trainings-Eingabewerten angewandt und muss entsprechende, vorher bekannte Trainings-Ausgabewerte erzeugen. Trainings-Eingabewerte sind erfindungsgemäß die Kalibrierdatensätze bevorzugt bezüglich einer Vielzahl von Testobjekten, deren theoretisches Röntgenschwächungsverhalten bekannt ist. Trainings-Ausgabewerte sind die entsprechenden, idealen Projektionsmessdatensätze, die bspw. aus einer Trainingsdatenbank nach Bedarf abgerufen werden können. Iterativ werden mittlere quadratische Fehler (mean square error - "MSE") zwischen berechneten und erwarteten Ausgabewerten berechnet und einzelne Gewichtungsfaktoren 420 so lange angepasst, bis die Abweichung zwischen berechneten und erwarteten Ausgabewerten unterhalb einer vorbestimmten Schwelle liegt.

**Figur 3** zeigt den schematischen Aufbau eines zählenden, bzw. direkt-konvertierenden Röntgendetektors 9 in einem Ausführungsbeispiel. Dieser besteht aus einer Vielzahl von Detektormodulen 35. Ein Detektormodul 35 umfasst eine Vielzahl von Pixelelementen 15, ein ASIC 27, einen Abschnitt eines direkt-konvertierenden Materials bzw. Direktkonverters 24 und eine Kopplung zwischen Direktkonverter 24 und ASIC 27 (bspw. Bumpbonds 36). Der ASIC 27 ist über ein Substrat 37 mit peripherer Elektronik 38 verbunden. Einfallende Röntgenstrahlung wird im Direktkonverter 24 (bspw. CdTe oder CZT) konvertiert und die erzeugten Ladungsträgerpaare über ein elektrisches Feld, welches von einer gemeinsamen Top-Elektrode 26 und einer für jedes Pixelelement 15 spezifischen Pixelelektrode 25 erzeugt wird, separiert. Die Ladung erzeugt in einer der Pixelelektroden 25 des ASIC 27 einen Ladungspuls, dessen Höhe der Röntgenquantenenergie entspricht. In einem Pixelelement 125 wird also ein Ereignis gezählt, indem eine digitale Speichereinheit um Eins hochgezählt wird. Liegt das erzeugte elektrische Signal oberhalb eines von mindestens einem einstellbaren Schwellwert, wird die Speichereinheit des darüber liegenden Energiebands hochgezählt, ansonsten wird die Speichereinheit des darunter liegenden Energiebands hochgezählt. Die in einem bestimmten Energiebereich bzw. Energieband gezählten Röntgenquanten lassen sich durch Differenzbildung der Zählerinhalte zweier entsprechender Zähler erhalten. Ein Schwellwert wird mittels Diskriminator festgestellt. Der Schwellwert kann prinzipiell einerseits fest analog vorgegeben sein, wird aber i.A. über einen Digital-Analog-Wandler (digital-to-analog-converter, DAC) angelegt und ist damit in einem gewissen Bereich variabel einstellbar. Grundsätzlich kann ein Schwellwert pixelweise oder global für alle Pixelelemente 15 einstellbar sein. Der erfindungsgemäße Röntgendetektor weist wenigstens einen globalen Schwellwert auf.

**Figur 4** zeigt eine medizinische Bildgebungsanlage 1 in Form eines Computertomographen 1. Andere Röntgen-Bildgebungsanlagen sind ebenfalls denkbar. Die Bildgebungsanlage 1 ist ausgebildet, das erfindungsgemäße Korrekturverfahren durchzuführen. Insbesondere ist die Bildgebungsanlage 1 ausgebildet, einen medizinischen Eingabedatensatz EG und einen medizinischen Ausgabedatensatz AG zu erzeugen. Die Bildgebungsanlage 1 ist ferner ausgebildet, eine erfindungsgemäße Korrekturfunktion KF zu bestimmen.

Der Computertomograph 1 verfügt über eine Aufnahmeeinheit 17, umfassend eine Röntgen(strahlungs)quelle 8 sowie einen energieselektiven Röntgen(strahlungs)detektor 9. Der Röntgendetektor 9 ist als direkt-konvertierender Röntgendetektor 9 und bspw. im Wesentlichen so ausgebildet, wie mit Bezug zu Figur 3 beschrieben. Andere Ausführungen sind ebenfalls denkbar. Die Aufnahmeeinheit 17 rotiert während der Aufnahme von spektral separierten Röntgenprojektionen um eine Systemachse 5, und die Röntgenquelle 8 emittiert während der Aufnahme Röntgenstrahlen 2, diese passieren den Patienten 3 und werden dabei abgeschwächt und treffen als Projektionsdaten auf den Röntgendetektor 9.

Der Patient 3 liegt bei der Aufnahme der spektral separierten Röntgenprojektionsdaten auf einer Patientenliege 6. Die Patientenliege 6 ist so mit einem Liegensockel 4 verbunden, dass er die Patientenliege 6 mit dem Patienten 3 trägt. Die Patientenliege 6 ist dazu ausgelegt, den Patienten 3 entlang einer Aufnahmerichtung durch die Öffnung 10 der Aufnahmeeinheit 17 zu bewegen. Die Aufnahmerichtung ist in der Regel durch die Systemachse 5 gegeben, um die die Aufnahmeeinheit 17 bei der Aufnahme der Röntgenprojektionsdaten rotiert. In diesem Beispiel ist die Körperachse des Patienten 3 gleich der Systemachse 5. Bei einer Spiral-Aufnahme wird die Patientenliege 6 kontinuierlich durch die Öffnung 10 bewegt, während die Aufnahmeeinheit 17 um den Patienten 3 rotiert und Röntgenprojektionen aufnimmt. Damit beschreiben die Röntgenstrahlen 2 auf der Oberfläche des Patienten 3 eine Spirale. Der Patient 3 kann, muss aber bei der Untersuchung nicht bewegt werden.

Der Computertomograph 1 verfügt über eine Recheneinheit 12 in Form eines Computers, welcher mit einer Anzeigeeinheit 11, beispielsweise zur graphischen Anzeige von medizinischen Bildaufnahmen, hier in Form von Computer-Tomographieaufnahmen oder einem Steuermenü für die Bildgebungsanlage 1, sowie einer Eingabeeinheit 7 bspw. zur Eingabe von Steuerbefehlen verbunden ist.

Die Recheneinheit 12 steht mit der drehbaren Aufnahmeeinheit 17 über eine Schnittstelle 31 zum Datenaustausch in Verbindung. Andererseits können für den Patienten 3 aufgenommene Projektionsdaten als medizinische Eingabedaten für das erfindungsgemäße Verfahren oder eine Bildrekonstruktion mittels gängiger Rekonstruktionsverfahren an den Computer 12 übertragen werden. Die Verbindung 14 ist in bekannter Weise kabelgebunden oder kabellos realisiert.

Die Recheneinheit 12 in Form des Computers gemäß diesem Ausführungsbeispiel umfasst einen Datenverarbeitungseinheit 16. Die Datenverarbeitungseinheit 16 umfasst Mittel für die Ausführung von Schritten des erfindungsgemäßen Verfahrens. Die Datenverarbeitungseinheit 16 umfasst folglich eine Bestimmungseinheit 21 zum Bestimmen einer Korrekturfunktion KF. Daneben umfasst die Datenverarbeitungseinheit 16 auch eine Korrektureinheit 23, die eingerichtet ist, die Korrekturfunktion KF auf den medizinischen Eingabedatensatz EG anzuwenden. Beide Einheiten 21 und 23 können als getrennte Verarbeitungseinheiten, aber auch gemeinsam in einer Einheit ausgebildet sein. Im ersten Fall stehen die Einheiten 21 und 23 zumindest in Datenverbindung, die einer Netzwerkverbindung 14 entsprechen kann, um die durch die Einheit 21 ermittelte Korrekturfunktion KF zur Anwendung an die Einheit 23 zu übertragen.

Die Recheneinheit 12 kann ferner eine Rekonstruktionseinheit 32 umfassen, welche anhand der um Datenfehler korrigierte Ausgabedaten AG von der Korrektureinheit 23 empfängt und unter Anwendung eines an sich bekannten Rekonstruktionsverfahrens medizinisches Bilddaten des Patienten 3 erzeugt. Die Rekonstruktionseinheit 32 kann auch als Sub-Komponente der Datenverarbeitungseinheit 16 ausgebildet sein. Jedenfalls besteht eine Datenverbindung zwischen Korrektureinheit 23 und Rekonstruktionseinheit 32 in Form einer Netzwerkverbindung 14.

Die Schnittstelle 31 kann als Hardware- oder Software-Schnittstelle ausgebildet sein, bspw. als PCI-Bus, USB oder Firewire. Ein Datenaustausch erfolgt bevorzugt mittels einer Netzwerk-Verbindung 14. Das Netzwerk kann als local area network (LAN), bspw. Ein Intranet oder ein wide area network (WAN) ausgebildet sein. Die Netzwerkverbindung 14 ist bevorzugt kabellos ausgebildet, bspw. als wireless LAN (WLAN oder WiFi). Das Netzwerk kann eine Kombination aus verschiedenen Netzwerkbeispielen umfassen. Datenübertragung kann basierend auf einer Datenabfrage oder eigeninitiativ erfolgen. Datenübertragung zwischen zwei Einheiten bzw. Systemkomponenten kann bidirektional oder unidirektional erfolgen.

Die Bestimmungseinheit 21 kann mit einer externen (nicht gezeigt) oder internen Speichereinheit 22 in Datenaustausch stehen, bspw. um Tabellen in Bezug auf ideale Schwächungsdaten bzw. ideale Schwächungsmesswerte bzgl. Basismaterialkombinationen, Photo- und Compton-Wirkungsquerschnitten oder monochromatischen Wirkungsquerschnitten abzurufen. Die externe Speichereinheit kann bspw. ein Krankenhaus- oder Radiologieinformationssystem-Server (HIS oder RIS) oder ein Cloud-Speicher sein, der auf an sich bekannte Weise der Recheneinheit 12 insbesondere automatisch bspw. Tabelleneinträge zuführt oder auf Abruf bereitstellt.

Die Recheneinheit 12 kann mit einem computerlesbaren Datenträger 13 zusammenwirken, insbesondere, um durch ein Computerprogramm mit Programmcode ein erfindungsgemäßes Verfahren durchzuführen. Weiterhin kann das Computerprogramm auf dem maschinenlesbaren Datenträger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 12, und damit auch ihre Sub-Komponenten, kann in Form von Hard- oder in Form von Software ausgebildet sein. Beispielsweise ist die Recheneinheit 12 als ein sogenanntes FPGA (Akronym für das englischsprachige "Field Programmable Gate Array") ausgebildet oder umfasst eine arithmetische Logikeinheit. Die Recheneinheit 12, einzelne oder alle ihrer Sub-Komponenten können alternativ dezentral angeordnet sein, z.B. können einzelne Rechenschritte des Verfahrens in einem zentralen Rechenzentrum einer medizinischen Dienstleistungseinrichtung, z.B. ein Krankenhaus, oder in der Cloud ausgeführt werden. Hierbei sind insbesondere Daten- und Patientenschutz beim Datenaustausch zu berücksichtigen.

Die Recheneinheit 12 kann weiter alternativ als Subkomponente des Röntgendetektors 9 oder als (eigenständiger) Cloudbasierter Computer ausgebildet sein, wobei der Datenaustausch mit der Bildgebungsablage 1 und/oder dem Röntgendetektor 9 über eine sichere Internet-Verbindung erfolgt. Die Kommunikation erfolgt in einem bevorzugten Ausführungsbeispiel per DICOM-Standard, andere Standards bzw. Datenformate sind jedoch ebenfalls möglich.

In der hier gezeigten Ausführungsform ist in einem Speicher (22) der Recheneinheit 12 wenigstens ein Computerprogramm gespeichert, welches Verfahrensschritte des erfindungsgemäßen Verfahrens durchführt, wenn das Computerprogramm auf dem Computer 12 ausgeführt wird. Das Computerprogramm zur Ausführung von Verfahrensschritten des erfindungsgemäßen Verfahrens umfasst Programmcode. Weiterhin kann das Computerprogramm als ausführbare Datei ausgebildet sein und/oder auf einem anderen Rechensystem als dem Computer 12 gespeichert sein. Beispielsweise kann der Computertomograph 1 so ausgelegt sein, dass der Computer 12 das Computerprogramm zum Ausführen des erfindungsgemäßen Verfahrens über ein Intranet oder über das Internet in seinen internen Arbeitsspeicher lädt.

Zusammengefasst liegen die Vorteile der vorliegenden Erfindung vor allem darin, dass die Korrektur der Pile-Up Nichtlinearität und die Korrektur der Strahlaufhärtung nicht mehr voneinander separiert korrigiert werden müssen. Die erfindungsgemäße Lösung führt beide Korrekturen und ggf. noch die Streustrahlkorrektur mittels darauf angepasster Korrekturfunktion in einem Korrekturschritt aus. Viele Kalibriermessungen und/oder Simulationen werden kombiniert, um die Korrekturfunktion bzw. den Korrekturalgorithmus zu parametrieren. Ergebnis des erfindungsgemäßen Korrekturverfahrens sind Fehler-bereinigte, korrigierte Röntgenschwächungswerte, die direkt, also ohne weitere Vorverarbeitung - in eine Bildrekonstruktion einfließen können. Da die Röntgenschwächungswerte eines Projektionsdatensatzes typischerweise jeweils für wenigstens zwei Energiekanäle vorliegen, eignen sich die korrigierten Röntgenschwächungswerte zur Erstellung monochromatischer Bilder entsprechend einer gewünschten Röntgenenergie mittels Linearkombination.

**Patentansprüche**

1. Computerimplementiertes Verfahren zur Korrektur eines Eingabedatensatzes (EG), umfassend folgende Schritte:

   - Erfassen (S1) eines Eingabedatensatzes umfassend wenigstens einen Datenfehler,
   - Bestimmen (S2, S21, S22) einer Korrekturfunktion (KF),
   - Erzeugen (S3) eines korrigierten Ausgabedatensatzes (AG) durch Anwenden der Korrekturfunktion auf den Eingabedatensatz,
   - Ausgeben (S4) des korrigierten Ausgabedatensatzes,

   wobei das Bestimmen (S2, S21, S22) der Korrekturfunktion (KF) den Schritten des Erfassens (S1), des Erzeugens (S3) und des Ausgebens (S4) vorgelagert erfolgt, und wobei die eine Korrekturfunktion ausgebildet ist, eine Reduzierung von wenigstens zwei, sich gegenseitig beeinflussenden Datenfehlern, umfassend wenigstens Datenfehler basierend auf einem während der Erfassung des Eingabedatensatzes auftretenden Pile-Up-Effekt und Strahlaufhärtungseffekt, in dem Eingabedatensatz durch gemeinsame Korrektur zu bewirken.

2. Verfahren nach Anspruch 1, wobei die Korrekturfunktion ferner ausgebildet ist, einen weiteren Datenfehler basierend auf einem Streustrahlungseffekt in dem Eingabedatensatz zu reduzieren.

3. Verfahren nach einem der vorhergehenden Ansprüche, wobei die Korrekturfunktion eine von einem Monitorwert (m) bezüglich der für die Erfassung des Eingabedatensatzes verwendeten Röntgenstrahlenquelle (8) abhängige Funktion ist.

4. Verfahren nach einem der vorhergehenden Ansprüche, wobei ein Bestimmen der Korrekturfunktion eine Kalibriermessung mit einem spektral auflösenden Röntgendetektor (9) umfasst.

5. Verfahren nach Anspruch 4, wobei die Kalibriermessung ein Erfassen von Kalibrierdaten eines Testobjektes umfassend wenigstens zwei Basismaterialien umfasst.

6. Verfahren nach Anspruch 5, wobei die Basismaterialien eine der folgenden Materialkombinationen umfasst:

   - Wasser - Iod,
   - Wasser - Knochen,
   - Aluminium - Eisen.

7. Verfahren nach Anspruch 4, wobei ein Bestimmen der Korrekturfunktion eine Kalibriermessung eines Testobjektes berücksichtigend wenigstens zwei integrierte Wirkungsquerschnitte von Compton-Effekt und Photoeffekt umfasst.

8. Verfahren nach Anspruch 4, wobei ein Bestimmen der Korrekturfunktion eine Kalibriermessung eines Testobjektes berücksichtigend wenigstens zwei monochromatische Wirkungsquerschnitte umfasst.

9. Verfahren nach einem der Ansprüche 1 bis 3, wobei ein Bestimmen der Korrekturfunktion eine Simulation einer Kalibriermessung umfasst.

10. Verfahren nach einem der Ansprüche 1 bis 3, wobei die Korrekturfunktion als eine mittels maschinellem Lernen trainierte Funktion (400) ausgebildet ist.

11. Recheneinheit (12) zur Korrektur eines Eingabedatensatzes (EG), umfassend:

    - eine Schnittstelle (31) eingerichtet zum Erfassen eines Eingabedatensatzes umfassend wenigstens einen Datenfehler,
    - eine Bestimmungseinheit (21) eingerichtet zum Bestimmen einer Korrekturfunktion (KF) vor dem Erfassen, dem Erzeugen und dem Ausgeben,
    - eine Korrektureinheit (23) eingerichtet zum Erzeugen eines korrigierten Ausgabedatensatzes (AG) durch Anwenden der Korrekturfunktion auf den Eingabedatensatz, und
    - eine Schnittstelle eingerichtet zum Ausgeben des korrigierten Ausgabedatensatzes,

    wobei die eine Korrekturfunktion ausgebildet ist, eine Reduzierung von wenigstens zwei, sich gegenseitig beeinflussenden Datenfehlern, umfassend wenigstens Datenfehler basierend auf einem während der Erfassung des Eingabedatensatzes auftretenden Pile-Up-Effekt und Strahlaufhärtungseffekt, in dem Eingabedatensatz durch gemeinsame Korrektur zu bewirken.

12. Röntgendetektor (9) in Form eines energieselektiven Röntgendetektors, umfassend eine Recheneinheit (12) gemäß Anspruch 11.

13. Computerprogramm, welches direkt in eine Speichereinheit (22) einer Recheneinheit (12) ladbar ist, mit Programmabschnitten, um Schritte des Verfahrens nach einem der Ansprüche 1 bis 10 auszuführen, wenn das Computerprogramm in der Recheneinheit (12) ausgeführt wird.

14. Computerlesbares Medium, auf welchem von einer Recheneinheit (12) einlesbare und ausführbare Programmabschnitte gespeichert sind, um Schritte des Verfahrens nach einem der Ansprüche 1 bis 10 auszuführen, wenn die Programmabschnitte von der Recheneinheit (12) ausgeführt werden.

## Claims

1. Computer-implemented method for correction of an input dataset (EG), comprising the following steps:

    - acquisition (S1) of an input dataset comprising at least one data error,
    - determination (S2, S21, S22) of a correction function (KF),
    - creation (S3) of a corrected output dataset (AG) by application of the correction function to the input dataset,
    - outputting (S4) of the corrected output dataset,

    wherein the determination (S2, S21, S22) of the correction function (KF) precedes the steps of acquiring (S1), creating (S3) and outputting (S4), and wherein the one correction function is embodied to bring about a reduction of at least two data errors that mutually influence one another in the input dataset, comprising at least data errors based on a pile-up effect and beam hardening effect occurring during the acquisition of the input dataset, by mutual correction.

2. Method according to claim 1, wherein the correction function is further embodied to reduce a further data error based on a scattered radiation effect in the input dataset.

3. Method according to one of the preceding claims, wherein the correction function is a function dependent on a monitor value (m) relating to the x-ray source (8) used for the acquisition of the input dataset.

4. Method according to one of the preceding claims, wherein a determination of the correction function comprises a calibration measurement with a spectrally resolving x-ray detector (9).

5. Method according to claim 4, wherein the calibration measurement comprises an acquisition of calibration data of a test object comprising at least two base materials.

6. Method according to claim 5, wherein the base materials comprise one of the following material combinations:

    - Water - iodine,
    - Water - bone,
    - Aluminium - iron.

**7.** Method according to claim 4 wherein a determination of the correction function comprises a calibration measurement of a test object taking into account at least two integrated cross sections of Compton effect and photo effect.

**8.** Method according to claim 4, wherein a determination of the correction function comprises a calibration measurement of a test object taking into account at least two monochromatic cross sections.

**9.** Method according to one of claims 1 to 3, wherein a determination of the correction function comprises a simulation of a calibration measurement.

**10.** Method according to one of claims 1 to 3, wherein the correction function is embodied as a function (400) trained by means of machine learning.

**11.** Computing unit (12) for correction of an input dataset (EG), comprising:

- an interface (31) configured for acquisition of an input dataset comprising at least one data error,
- a determination unit (21) configured for determining a correction function (KF) before acquisition, creation and output,
- a correction unit (23) configured for creating a corrected output dataset (AG) by applying the correction function to the input dataset, and
- an interface configured for outputting the corrected output dataset,

wherein the correction function is embodied to bring about a reduction of at least two data errors in the input dataset that mutually influence one another, comprising at least data errors based on a pile-up effect and beam hardening effect occurring during the acquisition of the input dataset, by mutual correction.

**12.** X-ray detector (9) in the form of an energy-selective x-ray detector, comprising a computing unit (12) according to claim 11.

**13.** Computer program, which is able to be loaded directly into a memory unit (22) of a computing unit (12), with program sections for carrying out steps of the method according to one of claims 1 to 10 when the computer program is executed in the computing unit (12).

**14.** A computer-readable medium, on which program sections able to be read in and executed by a computing unit (12) are stored, for carrying out steps of the method according to one of claims 1 to 10 when the program sections are executed by the computing unit (12).

**Revendications**

**1.** Procédé mis en œuvre par ordinateur de correction d'un ensemble (EG) de données d'entrée comprenant les stades suivants :

- saisie (S1) d'un ensemble de données d'entrée comprenant au moins une erreur de données,
- détermination (S2, S21, S22) d'une fonction (KF) de correction,
- production (S3) d'un ensemble (AG) de données de sortie corrigé par application de la fonction de correction à l'ensemble de données d'entrée,
- sortie (S4) de l'ensemble de données de sortie corrigé,

dans lequel la détermination (S2, S21, S22) de la fonction (KF) de correction s'effectue en étant placée avant les stades de la saisie (S1), de la production (S3) et de la sortie (S4), et dans lequel la une fonction de correction est constituée pour provoquer, par correction conjointe dans l'ensemble de données d'entrée, une réduction d'au moins deux erreurs de données s'influençant mutuellement, comprenant au moins une erreur de donnée reposant sur un effet de pile-up se produisant pendant la saisie de l'ensemble de données d'entrée et un effet de durcissement du faisceau.

**2.** Procédé suivant la revendication 1, dans lequel la fonction de correction est constituée en outre pour réduire, dans l'ensemble de données d'entrée, une autre erreur de donnée reposant sur un effet de rayonnement de dispersion.

**3.** Procédé suivant l'une des revendications précédentes, dans lequel la fonction de correction est une fonction, qui dépend d'une valeur (m) de monitor se rapportant à la source (8) de rayonnement X utilisé pour la saisie de l'ensemble de données d'entrée.

**4.** Procédé suivant l'une des revendications précédentes, dans lequel une détermination de la fonction de correction comprend une mesure d'étalonnage par un détecteur (9) de rayons X à résolution spectrale.

**5.** Procédé suivant la revendication 4, dans lequel la mesure d'étalonnage comprend une saisie de données d'étalonnage d'un objet test comprenant au moins deux matériaux de base.

**6.** Procédé suivant la revendication 5, dans lequel les matériaux de base comprennent l'une des combinaisons de matériaux suivants :

- eau - iode,
- eau - os,
- aluminium - fer.

7. Procédé suivant la revendication 4, dans lequel une détermination de la fonction de correction comprend une mesure d'étalonnage d'un objet test se rapportant à au moins deux sections efficaces intégrées d'effet Compton et de photo-effet.

8. Procédé suivant la revendication 4, dans lequel une détermination de la fonction de correction comprend une mesure d'étalonnage d'un objet test se rapportant au moins à deux sections efficaces monochromatiques.

9. Procédé suivant l'une des revendications 1 à 3, dans lequel une détermination de la fonction de correction comprend une simulation d'une mesure d'étalonnage.

10. Procédé suivant l'une des revendications 1 à 3, dans lequel la fonction de correction est constituée sous la forme d'une fonction (400) ayant subi un apprentissage au moyen d'un enseignement automatique.

11. Unité (12) informatique de correction d'un ensemble (EG) de données d'entrée, comprenant :

    - une interface (31) agencée pour la saisie d'un ensemble de données d'entrée comprenant au moins une erreur de donnée,
    - une unité (21) de détermination agencée pour la détermination d'une fonction (KF) de correction avant la saisie, la production et la sortie,
    - une unité (23) de correction agencée pour la production d'un ensemble (AG) de données de sortie corrigé par application de la fonction de correction à l'ensemble de données d'entrée, et
    - une interface agencée pour la sortie de l'ensemble de données de sortie corrigé,

    dans laquelle la fonction de correction est constituée pour provoquer dans l'ensemble de données d'entrée, par correction conjointe, une réduction d'au moins deux erreurs de données s'influençant mutuellement, comprenant au moins une erreur de donnée reposant sur un effet de pile-up se produisant pendant la saisie de l'ensemble de données d'entrée et un effet de durcissement du faisceau.

12. Détecteur (9) de rayons X sous la forme d'un détecteur de rayons X sélectif en énergie, comprenant une unité (12) informatique suivant la revendication 11.

13. Programme d'ordinateur, qui peut être chargé directement dans une unité (22) de mémoire d'une unité (12) informatique, comprenant des parties de programme pour exécuter les stades du procédé suivant l'une des revendications 1 à 10, lorsque le programme d'ordinateur est exécuté dans l'unité (12) informatique.

14. Support, déchiffrable par ordinateur, sur lequel sont mises en mémoire des parties de programme pouvant être lues et exécutées par une unité (12) informatique, afin d'exécuter les stades du procédé suivant l'une des revendications 1 à 10, lorsque les parties de programme sont exécutées par l'unité (12) informatique.

## FIG 1

S1

EG

S21

S2 { KD/KF

S22

SIMKD/
KF

S3

AG

S4

FIG 2

410   420   430   400   440

$x_1$ → $w_1$

$f\left(\sum x_i w_{i1}\right) = h_1$
$f\left(\sum x_i w_{i1}\right) = h_{1n}$
$f\left(\sum x_i w_{i1}\right) = h_{1n}$

$x_2$ → $w_2$

$f\left(\sum x_i w_{i2}\right) = h_2$
$f\left(\sum x_i w_{i2}\right) = h_{2n}$
$f\left(\sum x_i w_{i2}\right) = h_{2n}$

$x_3$ → $w_3$

$f\left(\sum x_i w_{i3}\right) = h_3$
$f\left(\sum x_i w_{i3}\right) = h_{3n}$
$f\left(\sum x_i w_{i3}\right) = h_{3n}$

$x_i$ → $w_i$

$f\left(\sum x_i w_{ij}\right) = h_j$
$f\left(\sum x_i w_{ij}\right) = h_{jn}$

$f\left(\sum w_{ij}' h_i\right) = o_j$
$f\left(\sum w_{ij}' h_i\right) = o_{jn}$

→ $o_j$

EP 3 798 684 B1

FIG 3

FIG 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006094493 A

- US 2017224299 A